(19) Europäisches Patentamt
European Patent Office
Office européen des brevets

(11) Publication number: 0 164 586 B1

(12) EUROPEAN PATENT SPECIFICATION

(45) Date of publication of patent specification: 29.06.88

(51) Int. Cl.⁴: C 12 Q 1/68 // G01N33/534, G01N33/60

(21) Application number: 85105792.7

(22) Date of filing: 11.05.85

# (54) Nucleic acid probe coupled to radioactive label.

(30) Priority: 23.05.84 US 612983

(43) Date of publication of application: 18.12.85 Bulletin 85/51

(45) Publication of the grant of the patent: 29.06.88 Bulletin 88/26

(84) Designated Contracting States: DE FR GB

(58) References cited:
EP-A-0 097 373
GB-A-2 055 104
GB-A-2 095 833
US-A-4 184 037
US-A-4 260 737
US-A-4 430 318

(73) Proprietor: Molecular Diagnostics, Inc.
400 Morgan Lane
West Haven, CT.06516 (US)

(72) Inventor: Dattagupta, Nanibhushan
470 Prospect Street
New Haven, CT. 06511 (US)

(74) Representative: Adrian, Albert, Dr. et al
c/o BAYER AG Konzernverwaltung RP
Patentabteilung
D-5090 Leverkusen 1, Bayerwerk (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 164 586 B1

## Description

Nucleic acid hybridization methods are widely used for the detection and isolation of specific genes. To date, radioactively labelled nucleic acid probes constitute the most sensitive detection system. The method of radioactive labelling includes the nick translation process, the use of kinase to phosphorylate the 5′ hydroxy residue and the use of terminal transferase (TdT). For oligonucleotides, the nick translation method is not applicable and the use of TdT (for labelling purposes) can produce heterogeneous distribution of molecules. Moreover, for restriction fragments, kinase mediated reaction will work only after the removal of existing 5′-phosphate by alkaline phosphatase. All these procedures can be practiced only by skilled workers in the art. After all the above mentioned reactions, difficult purifications and separation of the labelled probe from the enzyme and unreacted radioactive starting materials are still required.

In EP—A 63 879 an allylamin group linked to the base moiety of a nucleic acid is described. EP—A 97 373 discloses coupling of labelled bases to a nucleic acid by using a terminal transferase.

It is accordingly an object of the present invention to provide a method of labelling oligonucleotide and single or double stranded DNA or RNA, via a nonenzymatic chemical method.

This and other objects and advantages are realized in accordance with the present invention pursuant to which a nucleic acid to serve as a probe is modified at the 3′ or 5′ end to form a nucleotide with a primary alkylamine residue. The primary amine is then chemically reacted with the radioactive molecule. The reaction can be of the following types:

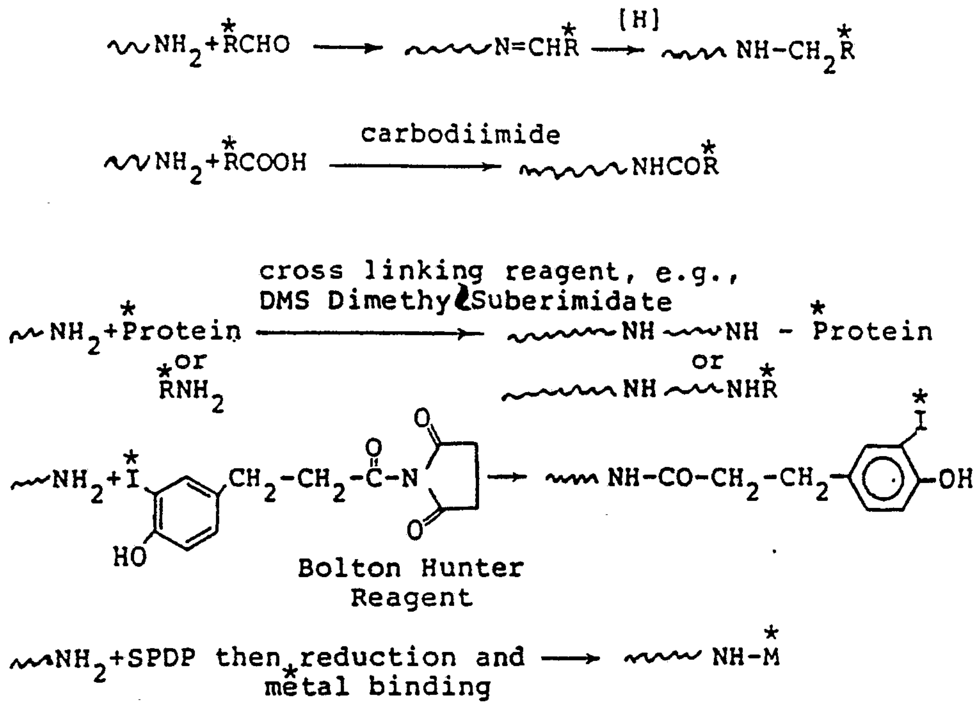


The new compounds invented for this purpose include nucleic acids with terminal nucleotides with —$NH_2$, —SH, —Hg. They are described schematically in the drawings wherein Figures 1 to 3 are schematic flow sheets of the synthesis and labelling of modified nucleic acid probes in accordance with the present invention.

Referring now more particularly to the drawings, in Fig. 1, a single or double stranded nucleic acid probe is enzymatically coupled to a ribonucleoside triphosphate residue.

With the bases, as in Figure 1, the product is subjected to periodate oxidation to split the cis-diol, forming two aldehyde groups which then condense with a diamine or polyamine as in Schiff's base condensation, followed by sodium borohydride reduction of the Schiff's base function to form the corresponding saturated amine. If the aldehyd groups are condensed with the amino group of an amino-alkylthioalcohol, the result is a probe which carries an —SH group.

In Fig. 2, (A) at the top, the products of Fig. 1 with free-$NH_2$ groups are reacted with oxidized α $^{32}$p-labelled ATP to product a Schiff's base which is borohydride-reduced to give a $^{32}$p-labelled product.

In Fig. 2(B), at the bottom, the products of Fig. 1 are directly reacted with Bolton Hunter reagent to form an amid carrying a radioactive $^{125}$I residue.

On the other hand, the nucleoside triphosphate may carry a Hg or SH radical, as on the purine or pyrimidine ring.

The invention will be further described in the following illustrative examples wherein all parts are by weight unless otherwise expressed:

Example 1

*Preparation of modified oligonucleotide with a spermine residue at the 3' end.*

HB 19 A' or HB 19S (Conner et al, Proc. Natl. Acad. Sic., U.S., *80,* 278 (1983)) have been prepared by a known triester method (Dembek et al., J. Am. Chem. Soc., *103,* 706 (1981). 40 µg of the oligonucleotide is dissolved in 100 µl TdT buffer (potassium cacodylate 200 mM (pH 7.2); 5 mM magnesium chloride; 0.2 mM dithiothreitol). To the solution, 10 µl 1mM ATP is added. The mixture is incubated at 37°C for 5 minutes. Then 400 units of terminal deoxynucleotidyl transferase (TdT) is added. (TdT was purchased from P. L. Biochemicals). The reaction mixture is incubated at 37°C for 45—60 minutes, then quenched by adding EDTA (final concentration 10 mM) and warming the solution to 60°C. The pH of the solution is then raised to 12—13 by adding 1M sodium hydroxide. The whole solution is then heated at 60°C for one hour. This procedure removes all but one terminal ribonucleotide residue. The solution is then brought to pH 9 with hydrochloric acid and 10 units of bacterial alkaline phosphatase is added. The mixture is incubated at 37°C for 10 hours and then neutralized with HCl to pH 7. The solution is then diluted to about 1 ml (10 times dilution) with water. This reduces the ionic strength of the solution. The mixture is then loaded onto a DEAE cellulose column (5 cm length, 0.5 cm diameter) which has been pre-equilibriated with TE (10 mM Tris, 1 mM EDTA; pH 7.2, adjusted with hydrochloric acid). The column is then washed with TE buffer containing 100 mM NaCl. Oligonucleotide with the terminal ribonucleotide residue is eluted with TE containing 600 mM NaCl. The nucleic acid sample is then partially desalted on a Sephadex G10 column. The final volume of solution containing the nucleic acid is about 300 µl. To this solution, acetic acid (1M) is added to adjust the pH to 5 and then a freshly prepared solution of sodium periodate (pH 5, Na-acetate buffer) is added to a final concentration of 1 mM. The oxidation is allowed to proceed for 30 min at 25°C. Then the pH of the solution is increased to 8.5 with sodium hydroxide and one tenth volume of 10 mM hexamethylene diamine or spermine solution (pH adjusted to 7—8 for solubilization) in water is added. The reaction mixture is incubated at 25°C for 60 minutes and the Schiff's base formed is reduced with sodium borohydride by adding 10 mg borohydride solid in five steps. A 15 minute time interval is allowed between each step.

A similar procedure is followed for DNA. The exception is in the use of phenol extraction and ethanol precipitation for deproteinization. No DEAE column has been used when DNA is modified.

Summarizing the steps used:

Summarizing the steps used:

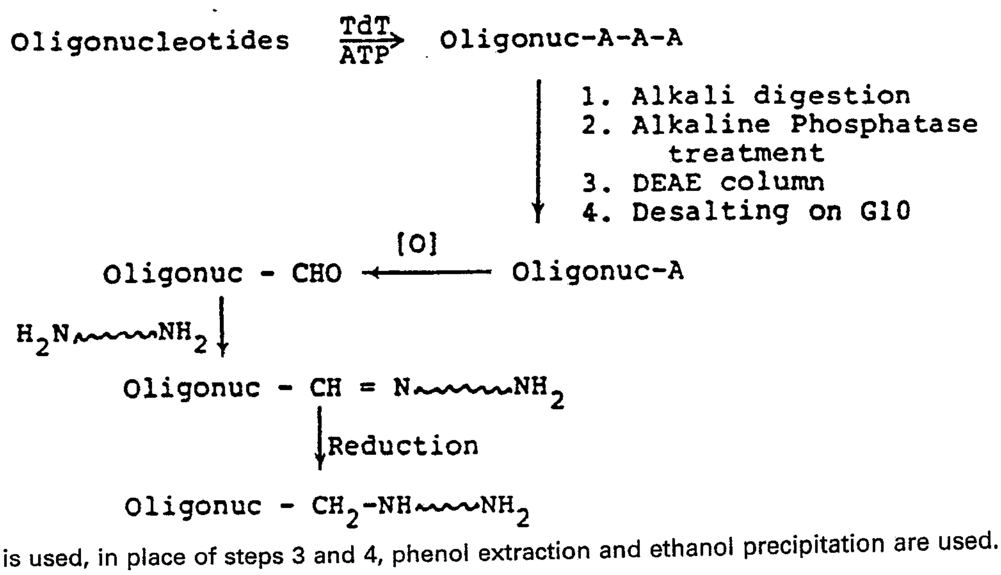


If DNA is used, in place of steps 3 and 4, phenol extraction and ethanol precipitation are used.

Example 2

*Coupling of hexylamino ATP to DNA or oligonucleotide at the 3' end.*

Nucleic acid sample as in example 1 is taken in TdT buffer. To the solution, 10 µl 1 mM hexylamino ATP is added. Then the TdT reaction is carried out as in example 1. Following the reaction, the sample is treated with 1M sodium hydroxide at 60°C for 30 minutes. Then it is neutralized with HCl to pH 7.2. For DNA, phenol extraction and ethanol precipitation is done by shaking the mixture with phenol, removing the phenol phase, adding ethanol and cooling the mixture on dry ice. For oligonucleotide, a DEAE column is used as in example 1. The same procedure can be followed to couple any —$NH_2$ containing nucleotide to DNA or oligonucleotide provided the residue is covalently linked to the nucleotide base in proper position.

Example 3

*Reaction of the product of Example 1 with BOLTON HUNTER REAGENT for radio iodination.*

Since DNA is not structurally affected by benzene, the aqueous solution of the product of example 1 (10—100 ug in 100 ul of 0.1 M borate buffer, pH 8) is mixed with 1mCi — Bolton Hunter reagent in the benzene solution supplied and the mixture is shaken for 15 minutes at 0°C. The reaction works at any temperature between 0 and 50°C and at pH between 7 and 9.5. At higher pH and higher temperature, the life time of the reagent is short and it reacts faster with water; hence the yield can go down to 10—15%. After the reaction, the whole mixture is passed through a Sephadex G25 column and the eluted radioactive fractions are cooled for hybridization purposes.

Example 4

*$^{32}P$ labelling without the use of an enzyme.*

$^{32}P$ labelled ATO (at the α-phosphate residue) is dissolved in 100 mM sodium acetate (pH 5) buffer (1 mM concentration) and oxidized with sodium periodate in conventional manner by adding sodium periodate (final concentration 2 mM). After the reaction, the pH of the solution is adjusted to 8 with sodium hydroxide solution.

Then the product is mixed with the product of Example 1 or 2 in the same buffer in equimolar concentration with respect to primary amine containing ends and the reaction is carried out for 60 minutes at 20°C. The product is reduced with sodium borohydride solution as in Example 1 and purified on a Sephadex G10 column, collecting only the fraction corresponding to void volume.

Example 5

*Hybridization of the labelled probe.*

The detection probe of EP 841 07 248.1 (EP—A 130515) is labeled with either $^{125}I$ or $^{32}P$ as in Examples 3 and 4, then hybridized with DNA for blood samples and assayed as described in the foregoing application.

Example 6

*Hybridization with labelled modified oligonucleotides.*

Modified oligonucleotides prepared from Hβ 19A′ and H 19S (Example 1) are labelled with $^{32}P$ as in example 4. The labelled modified oligonucleotides have been hybridized with blood DNA following the method of Conner et al (Proc. Natl. Acad. Sci., U.S. *80,* 278—282 (1983). The improvement in using the modified oligonucleotides is the labelling procedure. The present labelling method does not require an enzyme and the efficiency is greatly increased (30—90%).

**Claims**

1. A process for the production of a probe comprising a nucleic acid moiety covalently coupled at an end to a radioactive label through an —S—S, —Hg—S—, —CONH— or —$CH_2N$<linkage, carried by the terminal ribose residue said process comprises that

a) a nucleic acid which carries a ribose ring at the end is subjected to a periodate oxidation to split the cis-diol,

b) forming two aldehyd groups, followed by a condensation with a diamin, a polyamin or an amino-alkylthioalcohol, optionally followed by a sodium borohydrid reduction of the Schiff's base function and

c) the product of said condensation is a probe which carries an —$NH_2$ or —SH moiety which is coupled to radioactive material.

2. The process according to claim 1 wherein the probe carries an —$NH_2$ moiety which is coupled to a radioactive ATP.

3. The process according to claim 2 wherein the radioactive ATP is $^{32}P$-ATP.

4. The process according to claim 1 wherein the probe carries an —$NH_2$ moiety which is coupled to radioactive Bolton-Hunter reagent.

5. The process according to claim 4 wherein the Bolton-Hunter reagent carries a $^{125}J$ residue.

6. The process according to claim 1 wherein the probe carries a —SH residue which is coupled to a $^{35}S$ containing moiety.

7. The process according to claim 6 wherein the $^{35}S$ containing moiety is $^{35}S$-mercapto-ethanol.

8. Use of a probe produced by the process according to any of claims 1 to 7 in a nucleic acid hybridization method.

**Patentansprüche**

1. Verfahren zur Herstellung einer Sonde von einer Nucleinsäuregruppe, die kovalent an ein Ende einer radioaktiven Markierung mittels einer —S—S, —Hg—S—, —CONH— oder —$CH_2N$< Bindung, die an dem endständigen Riboserest vorhanden ist, gekuppelt ist, dadurch gekennzeichnet, dass man

(a) eine Nucleinsäure, die an dem Ende einen Ribosering trägt, einer Perjodatoxidation unter Aufsplittung des cis-Diols unterwirft,

(b) zwei Aldehydruppen bildet, anschliessend mit einem Diamin, einem Polyamin oder einem Aminoalkylthioalkohol kondensiert, gewünschtenfalls die Schiff'sche Basenfunktion mittels Natriumborhydrid reduziert und

(c) das Produkt der Kondensation eine Sonde ist, die eine —$NH_2$— oder —SH-Gruppe, die an das radioaktive Material gebunden ist.

2. Verfahren gemäss Anspruch 1, bei dem die Sonde eine an ein radioaktivs ATP gebundene —$NH_2$-Gruppe enthält.

3. Verfahren gemäss Anspruch 2, bei dem das radioaktive ATP $^{32}$P-ATP ist.

4. Verfahren gemäss Anspruch 1, bei dem die Sonde eine —$NH_2$-Gruppe enthält, die an ein radioaktives Bolton-Hunter-Reagens gekuppelt ist.

5. Verfahren gemäss Anspruch 4, bei dem das Bolton-Hunter-Reagens einen $^{125}$J-Rest enthält.

6. Verfahren gemäss Anspruch 1, bei dem die Probe einen —SH-Rest, der an einer $^{35}$S enthaltende Gruppe gekuppelt ist, enthält.

7. Verfahren gemäss Anspruch 6, bei dem die $^{35}$S enthaltende Gruppe $^{35}$S-Mercapto-ethanol ist.

8. Verwendung einer nach dem Verfahren gemäss einem der Ansprüche 1 bis 7 hergestellten Sonde in einem Nucleinsäure-Hybridisierungsverfahren.

**Revendications**

1. Procédé de production d'une sonde comprenant une portion d'acide nucléique dont le couplage par covalence à une extrémité à un marqueur radioactif est effectué par l'intermédiaire d'une liaison —S—S, —Hg—S—, —CONH— ou —$CH_2N$<, portée par le résidue ribose terminal, ledit procédé consistant

a) à soumettre un acide nucléique qui porte un cycle ribose à son extrémité à une oxydation par le periodate afin de scinder le diol en position cis,

b) à former deux groupes aldéhyde, puis à effectuer une condensation avec une diamine, une polyamine ou un aminoalkylthioalcool, suivie facultativement par une réduction par le borohydrure de sodium de la fonction base de Schiff et

c) à effectuer le couplage d'un groupement —$NH_2$ ou —SH porté par le produit de ladite condensation, qui constitue une sonde, à une matière radio-active.

2. Procédé suivant la revendication 1, dans lequel la sonde porte un groupement —$NH_2$ qui est couplé à un ATP radio-actif.

3. Procédé suivant la revendication 2, dans lequel l'ATP radio-actif est le $^{32}$P-ATP.

4. Procédé suivant la revendication 1, dans lequel la sonde porte un groupement —$NH_2$ qui est couplé à un réactif radio-actif de Bolton-Hunter.

5. Procédé suivant la revendication 4, dans lequel le réactif de Bolton-Hunter porte un résidu $^{125}$I.

6. Procédé suivant la revendication 1, dans lequel la sonde porte un résidu —SH qui est couplé à un groupement contenant un $^{35}$S.

7. Procédé suivant a revendication 6, dans lequel le groupement contenant un $^{35}$S est le $^{35}$S-mercapto-éthanol.

8. Utilisation d'une sonde produite par le procédé suivant l'une quelconque des revendications 1 à 7, dans un procédé d'hybridation d'acides nucléiques.

FIG. 1

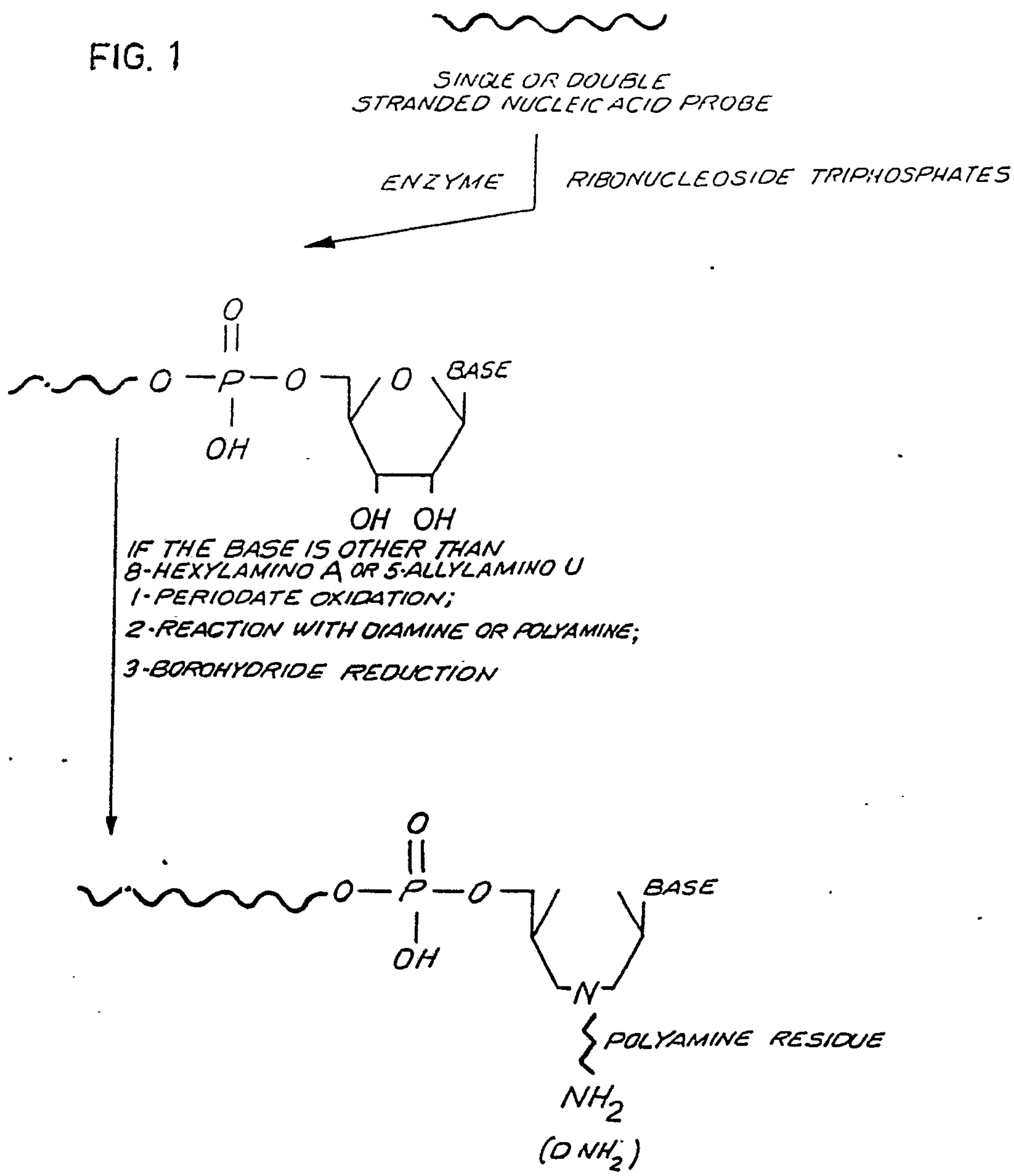


• FOR OLIGONUCLEOTIDE PROBE, IT CAN BE NON-ENZYMATICALLY SYNTHESIZED

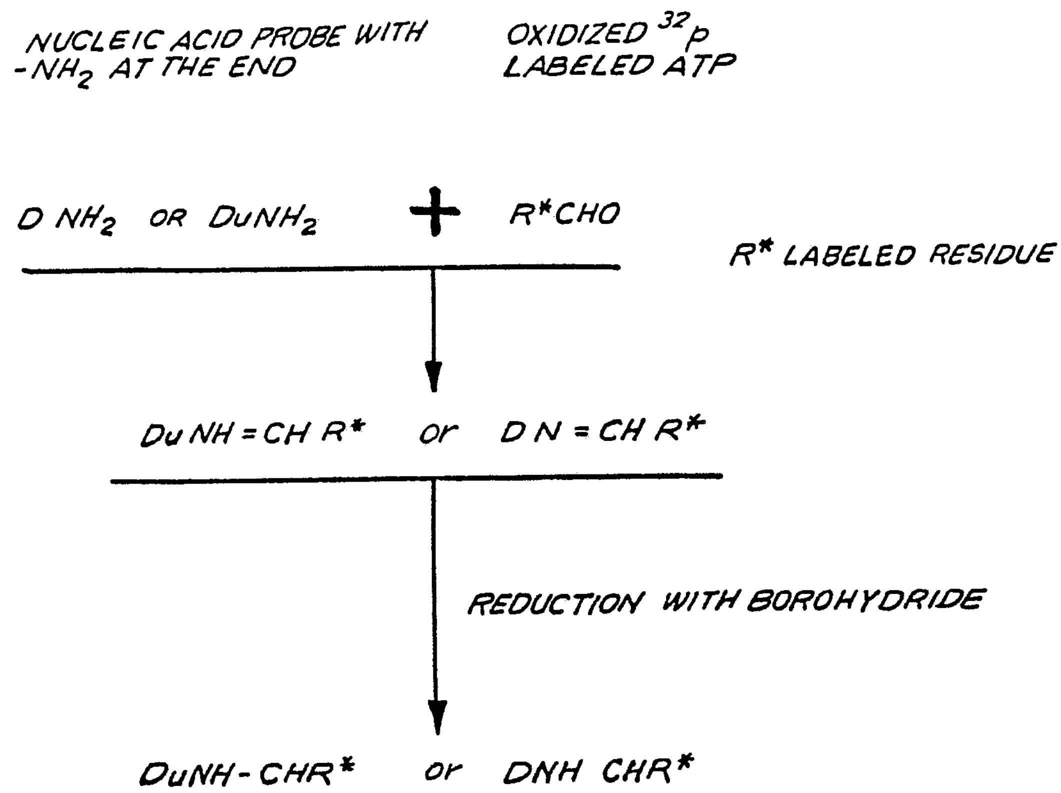
NUCLEIC ACID PROBE WITH -NH2 AT THE END
OXIDIZED 32P LABELED ATP
R* LABELED RESIDUE
REDUCTION WITH BOROHYDRIDE

FIG. 2a

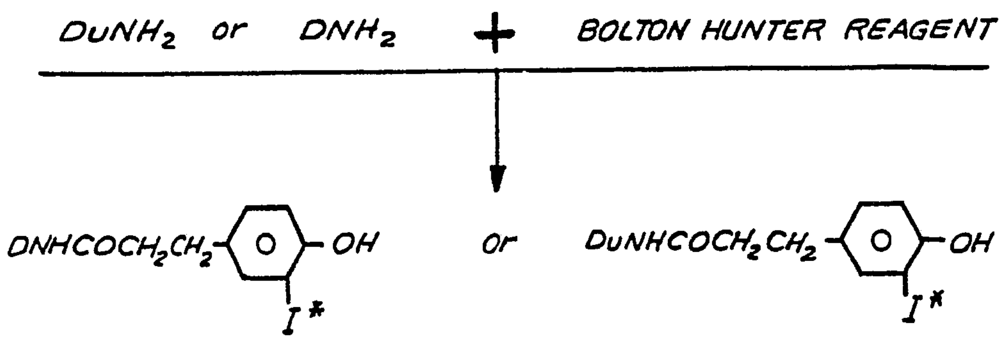
BOLTON HUNTER REAGENT

FIG. 2b